# EUROPEAN PATENT SPECIFICATION

(11) **EP 2 523 711 B1**
(45) Date of publication and mention of the grant of the patent: **09.08.2017**
(21) Application number: 11707711.5
(22) Date of filing: 12.01.2011
(51) Int. Cl.: A61M 11/00, A61M 11/02, A61M 11/06

(54) **A METHOD AND APPARATUS FOR PRODUCING FINE CONCENTRATED AEROSOL**
VERFAHREN UND VORRICHTUNG ZUR HERSTELLUNG EINES FEIN KONZENTRIERTEN AEROSOLS
PROCÉDÉ ET APPAREIL DESTINÉ À PRODUIRE UN AÉROSOL CONCENTRÉ FIN

(30) Priority: 12.01.2010 US 294161 P
(43) Date of publication of application: 21.11.2012
(73) Proprietor: Omega Life Science Ltd, 23101 Migdal Haemek (IL)
(72) Inventor: SADYKHOV, Akper, 26230 Kiryat Mozkin (IL)
(74) Representative: Meyer-Dulheuer, Karl-Hermann
(86) International application number: PCT/IL2011/000038
(87) International publication number: WO 2011/086552

(56) References cited:
- WO-A1-00/50111
- WO-A2-01/66064
- US-A- 5 301 664
- US-A- 5 915 378

## Description

### Field of invention

The present invention relates to a method and apparatus enabling production of ultra fine and concentrated aerosol from liquids, for various applications, by using rigid porous material, enabling storage and easy streaming of the aerosol, (i.e. for inhalation), fast replacement of liquid carrier device and accuracy of output sprayed dose.

### Background of invention

The efficiency and effectiveness of nebulizer technology depends on its ability to produce droplets of aerosol characterized by required parameters (i.e.: size, concentration etc.) since these parameters have a direct impact on the performance of the aerosolized material (i.e. therapeutic effect). Additional needs are to eliminate product loss during operation and to allow convenience of use.

From existing professional bibliography it is known that: Effective lung healing treatment requires a certain dose of medicine composed of particles sized 0.5 to 2 micrometer.

### Prior art

**U.S Patents**

| Patent Number | | Date of Patent |
|---|---|---|
| 4,743,407 | Externally pressurized porous cylinder for multiple surface aerosol generation and method of generation | May 10, 1988 |
| 6530370 | Nebulizer apparatus | Mar 11, 2003 |
| 5718222 | Disposable package for use in aerosolized delivery of druqs | Feb 17, 1998 |
| 6070575 | Aerosol-forming porous membrane with certain pore structure | Jun 6, 2000 |
| 6230706 | Method and device for creating aerosol with porous membrane with certain pore structure | May 15, 2001 |
| 5497763 | Disposable package for intrapulmonary delivery of aerosolized formulations | Mar 12, 1996 |
| 5855564 | Aerosol extrusion mechanism | Jan 5, 1999 |
| 7013894 | Portable, handheld, pneumatic driven medicinal nebulizer | Mar 21, 2006 |
| 5379760 | Position insensitive low resistance aspirator | Jan 10, 1995 |
| 6899322 | Method and apparatus for production of droplets | Dec 31, 2001 |
| 7562656 | Aerosol medication inhalation system | Sep 17, 2004 |
| 3762409 | Nebulizer | Nov 3, 1970 |
| D471626 | Nebulizer | : Dec 10, 2001 |
| RE30046 | Nebulizer | Jul 17, 1979 |
| 5823179 | Nebulizer Apparatus and method | Oct 20, 1998 |
| 5570682 | Passive aspiratory nebulizer system | Nov 5, 1996 |
| 5685291 | Nebulizer adapter system for premature babies | Nov 11, 1997 |

### Existing nebulizers:

Pneumatic nebulizers have orifices of 500 micrometer and more and other means of separation (extraction) of large droplets, therefore the obtained aerosol is of low concentration, (Concentration = Quantity of droplets per volume), usually less than 10^7/cm^3). Such low concentration increases the treatment time in order to achieve use of required dose. In addition there is a hardship to control the dose achieved by existing nebulizers, even some of the most advanced nebulizers entail a synchronization system between the aerosol supply and the respiration rhythm yet there is still aerosol loss and dose accuracy can hardly be maintained.

In the existing nebulizers a significant quantity of atomized liquid is lost in the process of preparing the aerosol due to "dead" volume that can never be sprayed as aerosol.

An additional disadvantage of the existing nebulizers is a mandatory use position (e.g: vertical) to allow proper device operation and to control the amount of the dose.

Another factor to be considered is the exposure of environment to the aerosol (i.e. medicine) while using the existing nebulizers. While patient uses a mask, tent or any other kind of disperser, there is an existing risk of medicine leakage that might be hazardous to environment.

To date the existing nebulizers of ultrasonic type do not allow spraying all kind of liquids since during their process the medicine absorbs ultrasonic energy and heats, which harms the medicine and can distort it. Such nebulizers require high rate supply of external air for liquid cooling and streaming thus creating a relatively low concentrated aerosol (even though higher than the pneumatic type nebulizers) but no more than 10^8/cm^3, in best conditions, therefore, the treatment time required, is still high.

The proposed technology resolves the above mentioned problems and overcomes the above encountered disadvantages while offering additional advantages.

### Summary of the invention

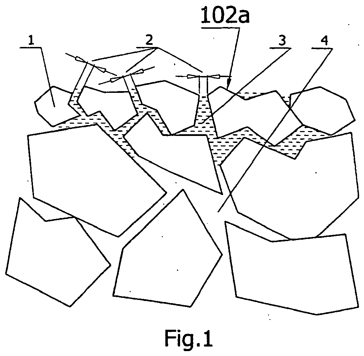

The proposed technology is intended for creating ultra fine aerosol by using rigid porous material (1) (fig.1).

The ultra fine aerosol (0.3 to 1.1 micrometer of droplet size of the aerosol) is achieved due to specific parameters of the porous material, while the porous material acts as a pneumatic multi-nozzle atomizing system. The porous medium itself is in fact an integral system consisting of the following elements:
- The liquid (3) for spraying (been on the surface of the porous medium and/or partially absorbed in it)
- A large number of pores (2) (of sub-micron size) acting as nozzles.
- Gas (4) caged in those pores which are vacant of liquid.

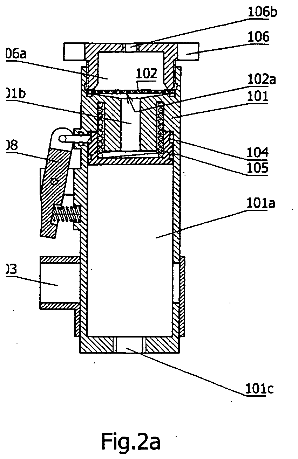

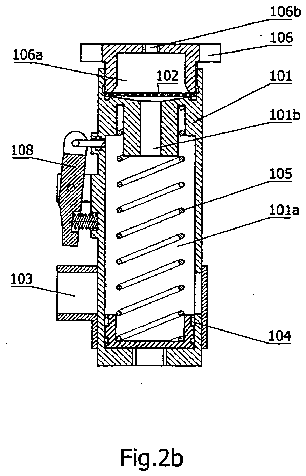

The aerosol spraying is performed by instantaneously inducing pressure drop to the thickness of the porous medium (for instance: when the medium is 'coin' shaped the differential pressure occurs between the two flat sides of the 'coin') . For example, when the porous medium (which contains the above mentioned liquid and gas) is in atmospheric pressure a sharp pressure drop is induced on one side of the medium (e.g. 600 to 900 mbar less than atmospheric pressure, depending on the porous medium characteristics) (Fig.2b) . In this stage atomizing occurs as a result of the differential pressure (between the two sides of the medium) which causes the medium to act as a pneumatic multi-nozzle. The reason for the effect of pneumatic multi-nozzle atomization is that the air (4) (Fig.1) which was caged in the internal volume of the porous medium releases itself in the direction of vacuum, causing the pores (2) (Fig. 1) to act as nozzles and spray the liquid (3) (Fig. 1) outside from the medium. The aerosol is then achieved on the side of the lower pressure (atomized side (102a)) (Fig2a) with no need for external (atomizing) gas supply.

The under-pressure mentioned above is produced in a chamber, whereas the porous medium is a part of the chamber.

According to the above technology, it is possible to achieve fine aerosol with high concentration (10^9-10^11 /cm^3) and more.

The same chamber in which the under-pressure (vacuum) is produced is also used for storing the aerosol, hereby referred to as "the vacuum accumulator", until the aerosol is required for use (i.e. inhalation), whereas the stability of the aerosol in the chamber depends on its concentration.

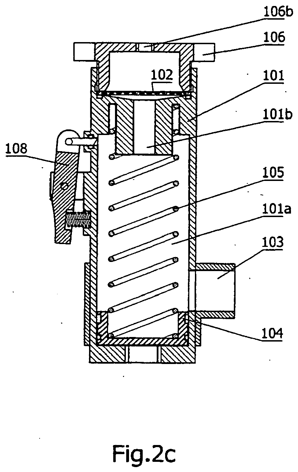

In order to take out aerosol (103) (Fig2c) from the vacuum accumulator, the pressure in the vacuum accumulator needs to be changed. For example, for an inhalator, an atmospheric pressure has to be produced in such a way that will allow the inhalation of the stored aerosol as a whole with no losses, dosed and repeatable, without need for any synchronization with the respiratory tract.

The vacuum accumulator element may also be used as a drying chamber depending on the relation between the volume of the accumulator chamber and the quantity of the droplet and on the overall parameters of the environment in vacuum accumulator (e.g. temperature, pressure, etc.). In such case dry aerosol can be obtained by the nebulizer (e.g. for purpose of dry particles inhalation, which is a new kind of dry-particle-inhaler that acts without pressurized gas).

Additional drying process can take place by the act of inhalation itself, when the inhaled air acts as drying agent.

The described nebulizer has the following advantages:
- The nebulizer can be operated in any environment and any position: upright, horizontal and even in outer space.
- Possible of being self sustained.

The porous element may also be used as a storage container for the liquid (i.e. medicine) prior to its conversion to aerosol (can be referred to as "pill"), when it is soaked with a determined quantity of liquid or alternatively when it is normally dry but covered with a buffering (dry) layer (that is not transferable for liquid) packed together with a liquid container (that together with the porous medium form a "sandwich" like device) that has a mechanism of wetting the medium (e.g. by removing the buffering layer) which is performed before the atomizing effect (before the insertion of the "pill" into the nebulizer, or afterwards by an internal mechanism of the nebulizer).

When carried around by user (i.e. prior to atomization process), the "pill" is hermetically and sterilely sealed and packed.

The "pill" is designed to be used with specific nebulizer device. Matching or unmatching combinations can be created to allow or deny certain usage combinations.

Serving as a liquid container the "pill" can contain any desired liquid formulation (e.g. medicine, food supplement, natural sources, etc.) while the "pill" serves as a generic platform for carrying the liquid, ready to be converted into aerosol anytime when fed into a nebulizer device (i.e. for inhalation purposes).

The "pill" can be disposable. It is designed for easy replacement and discharge.

### Description of the drawings:

Fig 1 describes: (1) - porosive material; (2) - nozzles (pores); (3) - liquid for spraying, (4) - gas
Figs .2a; 2b; 2c & 3 describe the nebulizer, in different stages: (2a) - when the nebulizer is in idle position, before aerosol is produced; (2b) - the nebulizer is in under-pressure production and aerosol production; (2c) - nebulizer during inhalation act.

### Detailed description:

- Outer structure (101) containing the vacuum accumulator (101a). The vacuum accumulator's dimensions are determined according to the volume of aerosol required for each application. For example: to produce 30 mg of medical aerosol for local delivery to the lung or systematic delivery through the lung, a volume of 30 cc is required.
- The vacuum accumulator has a path (101b) through which the aerosol received from the porous material (102) enters the vacuum accumulator (101a).
- The exit hole (103) for aerosol inhalation is externally closed by rotating cover.
- Under-pressure effect can be generated either externally by a vacuum pump (for stationary use) or by an internal device of the nebulizer itself, for example, with the help of the cylinder pistol (104) that is moved by spring (105).
- On the upper side of the device there is a place to insert the porous medium (102), which may be in the shape of a cylinder, disc, cup or the like, that if fixed or sealed to the device by cover (106), designed for fast opening/closing and has a duct (106b) connecting to environment or vacuum accumulator(101a).

The location of the porous media in relation to the device can be on its upper side (as appears in the drawing) but can also be in the opposite (bottom side) or in any side of the device.

The material, structure and dimensions of the porous material (102) are determined according to the required aerosol characteristics for each required application. For example: the dispersing area determines the quantity of achieved aerosol.

Fig. 2a shows the nebulizer prepared for generating aerosol:
- spring (105) is compressed;
- piston (104) is captured in upper position with fixture (108);
- the porous medium (102) inserts to a place and fixed by cover (106);
- the exit hole for aerosol closed by rotating cover (103).

Fig. 2b shows the spraying nebulizer :
- piston (104) is - in lower position;
- vacuum accumulator (101a) is filled by aerosol.

Fig. 2c shows the nebulizer prepared for aerosol exploitation: rotating cover (103) connecting to environment.

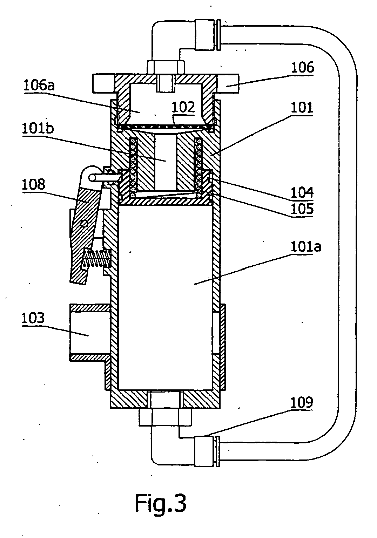

Fig 3 shows the nebulizer with tube (109) for supply air in spraying stage from vacuum accumulator (101a) to a duct (106b) to increase aerosol capacity by increasing dispersing air. Introducing additional dispersing air causes increase of the differential pressure.

## Claims

1. A porous medium (102) configured to act as a pneumatic multi-nozzle atomizing system which is designed for easy replacement and discharge from a nebulizer, wherein the porous medium (102) is a rigid material and has two flat sides and further comprises:
i. a plurality of pores (2),
ii. a liquid (3) partially adsorbed in the porous medium (102), ready to be converted into aerosol anytime when fed into the nebulizer; and
iii. gas (4), wherein the gas (4) is caged in pores (2) that are vacant of said liquid (3).

2. The porous medium of claim 1, wherein said liquid comprises a medication.

3. A nebulizer for producing aerosol, comprising the porous medium (102) of claim.

4. The nebulizer of claim 3, further comprising a chamber and a path,
wherein the path is configured to receive the aerosol from the porous medium and transfer said aerosol to the chamber; or
further comprising an outlet configured to release said aerosol by inhalation; or
further comprising an element configured for creating a pressure drop between the two sides of the porous medium, the element is selected from
a piston and spring, and a vacuum pump; or
further comprising cover, configured to open-close the nebulizer, the cover comprising a duct; or further comprising a tube connecting the chamber to said duct and configured to increase the pressure.

5. The nebulizer of claim 3, wherein the porous medium (102) is in a shape selected from the group consisting of: a cylinder, a disc and a cup.

6. The nebulizer of claim 3, wherein said liquid comprises a medication.

7. A method for producing ultra fine highly concentrated aerosol, comprising
a. providing the porous medium of claim 1; and
b. inducing a pressure drop between the two flat sides of the porous medium, thereby producing aerosol,
wherein the aerosol Is produced In the absence of an external gas supply.

8. The method of claim 7, wherein inducing the pressure drop is achieved by inducing a pressure drop on one side of said two flat sides of the porous medium; or
wherein inducing the pressure drop is achieved by a vacuum generating mean selected from a vacuum pump and a piston; or
further comprising storing the aerosol under vacuum; or
further comprising transferring the aerosol for storage under a vacuum in a chamber.

9. The method of claim 8, wherein pressure of the porous medium at step (a) is an atmospheric pressure and wherein said pressure drop reduces the atmospheric pressure in said one side by 600 to 900 mbar.

10. The method of claim 7, wherein the aerosol concentration is within the range of 10⁹ to 10¹¹ per cm³.

11. The method of claim 7, wherein the liquid comprises a medication.

## Patentansprüche

1. Ein poröses Medium (102), welches bei Zufuhr zum Zerstäuber sofort in Aerosol umgewandelt wird, ist so ausgelegt, dass es wie ein pneumatisches Mehrfachdüsen-Zerstäubungssystem, welches einfach ausgetauscht und vom Zerstäuber abgekoppelt werden kann, funktioniert, wobei das poröse Material (102) biegesteif ist, zwei flache Seiten hat und zusätzlich folgende Eigenschaften besitzt:
i. eine Vielzahl von Poren (2)
ii. Flüssigkeit (3) wird teilweise absorbiert von dem porösen Medium, welches bei Zufuhr zum Zerstäuber sofort in Aerosol umgewandelt wird; und
iii. Gas (4) in den Poren (2), die keine Flüssigkeit (3) enthalten.

2. Das poröse Medium nach Anspruch 1, wo besagte Flüssigkeit ein Medikament enthält.

3. Ein Zerstäuber für die Herstellung von Aerosol, der das poröse Medium (102) nach Anspruch 1 enthält, welches bei Zufuhr zum Zerstäuber sofort in Aerosol umgewandelt wird.

4. Der Zerstäuber nach Anspruch 3, der außerdem eine Kammer und einen derart ausgelegten Durchgang besitzt, dass das Aerosol vom porösen Medium aufgenommen und in die Kammer transportiert wird; oder
zusätzlich einen Ausgang besitzt, der besagtes Aerosol zur Inhalation freisetzt; oder zusätzlich ein für einen Druckabfall zwischen beiden Seiten des porösen Mediums ausgelegtes Element besitzt, welches von einem Kolben und einer Feder aktiviert wird, sowie eine Vakuumpumpe; oder
eine zusätzliche Abdeckung besitzt, durch die der Zerstäuber geöffnet und geschlossen wird, wobei auch diese Abdeckung einen Durchgang hat; oder
zusätzlich ein Rohr enthält, welches die Kammer mit besagtem Durchgang verbindet und damit den Druck erhöht.

5. Der Zerstäuber nach Anspruch 3 enthält ein poröses Medium (102), welches bei Zufuhr zum Zerstäuber sofort in Aerosol umgewandelt wird und der entsprechend der Auswahl aus einem Zylinder, einer Scheibe und einer Schale besteht.

6. Der Zerstäuber nach Anspruch 3, wobei die Flüssigkeit ein Medikament enthält.

7. Eine Methode zur Herstellung von ultrafeinem, hochkonzentrierten Aerosol, welche umfasst:
a. die Bereitstellung eines porösen Mediums wie in Anspruch 1 beschrieben; und
b. einen Druckabfall zwischen beiden Seiten des porösen Mediums, wodurch das Aerosol hergestellt wird,
wobei das Aerosol ohne zusätzliche Gaszufuhr hergestellt wird.

8. Die Methode nach Anspruch 7, wo ein Druckabfall durch den Druckabfall an einer der beiden Seiten des porösen Mediums erzeugt wird; oder
wo mittels einer Vakuumpumpe und eines Kolbens eines Vakuums erzeugt wird, welches einen Druckabfall generiert; oder
wo das Aerosol außerdem unter Vakuum gespeichert wird; oder
wo das Aerosol außerdem zur Speicherung in eine Vakuumkammer transportiert wird.

9. Die Methode nach Anspruch 8, wobei es sich hier beim Druck des porösen Mediums bei Schritt (a) um einen atmosphärischen Druck handelt und wobei besagter Druckabfall den atmosphärischen Druck an einer Seite um 600 mbar auf 900 mbar reduziert.

10. Die Methode nach Anspruch 7, wobei die Konzentration des Aerosols innerhalb von 10⁹ bis 10¹¹ pro cm³ liegt.

11. Die Methode nach Anspruch 7, wobei die Flüssigkeit ein Medikament enthält.

## Revendications

1. Un support poreux *(102)* configuré pour agir en tant que système atomiseur pneumatique à buses multiples, qui est conçu pour être facilement remplacé et détaché d'un nébuliseur, où le support poreux *(102)* est un matériau rigide et comporte deux faces planes et comprend par ailleurs :
i. une pluralité de pores *(2)*
ii. un liquide *(3)* partiellement adsorbé dans le support poreux *(102)*, prêt à être converti à tout moment en aérosol lorsqu'il est introduit dans un nébuliseur ; et
iii. un gaz *(4)*, où ce gaz est confiné dans des pores qui ne contiennent pas ledit liquide *(3)*.

2. Le support poreux de la revendication 1, où ledit liquide comprend un médicament.

3. Un nébuliseur servant à produire un aérosol ; comprenant le support poreux *(102)* de la revendication 1.

4. Le nébuliseur de la revendication 3, comprenant en outre une chambre et un conduit, où ce conduit est configuré de manière à recevoir l'aérosol provenant du support poreux et à transférer ledit aérosol vers la chambre ; ou
comprenant en outre une sortie configurée de manière à libérer ledit aérosol par inhalation ; ou
comprenant en outre un élément configuré de manière à créer une baisse de pression entre les deux faces du support poreux, l'élément est sélectionné parmi un piston et un ressort, et une pompe à vide ; ou
comprenant en outre un couvercle, configuré de manière à ouvrir-fermer le nébuliseur, *ce couvercle comprenant un conduit ; ou*
comprenant en outre un tube reliant la chambre audit conduit et configuré de manière à augmenter la pression.

5. Le nébuliseur de la revendication 3, où le support poreux (102) a une forme sélectionnée dans le groupe composé de : un cylindre, un disque et une cuvette.

6. Le nébuliseur de la revendication 3, où ledit liquide comprend un médicament.

7. Une méthode de production d'un aérosol ultra fin hautement concentré, comprenant
a. la fourniture du support poreux de la revendication 1 ; et
b. l'induction d'une baisse de pression entre les deux faces planes du support poreux, produisant ainsi un aérosol,
où l'aérosol est produit en l'absence d'un apport en gaz externe.

8. La méthode de la revendication 7, dans laquelle l'induction de la baisse de pression est obtenue en induisant une baisse de pression sur un côté desdites deux faces planes du support poreux; ou
dans laquelle l'induction de la baisse de pression est obtenue par un moyen de génération d'un vide sélectionné parmi une pompe à vide et un piston ; ou
comprenant en outre la conservation de l'aérosol sous vide ; ou
comprenant en outre le transfert de l'aérosol en vue de sa conservation sous vide dans une chambre.

9. La méthode de la revendication 8, dans laquelle la pression du support poreux à l'étape (a) est une pression atmosphérique et dans laquelle ladite baisse de pression réduit la pression atmosphérique dans ledit côté de 600 à 900 mbar.

10. La méthode de la revendication 7, dans laquelle la concentration de l'aérosol se situe dans la fourchette de 10⁹ à 10¹¹ par cm³.

11. La méthode de la revendication 7, dans laquelle le liquide comprend un médicament.
